# EUROPEAN PATENT SPECIFICATION

(11) **EP 2 075 257 B1**
(45) Date of publication and mention of the grant of the patent: **08.08.2012**
(21) Application number: 09003728.4
(22) Date of filing: 04.09.2003
(51) Int. Cl.: C07K 16/00, C07K 16/28, C12N 15/19, C12P 21/08

(54) **Preparation of antibody using mrl/lpr mouse**
Herstellung eines Antikörpers unter Verwendung einer MRL/LPR-Maus
Préparation d'anticorps au moyen d'une souris mrl/lpr

(30) Priority: 04.09.2002 WO PCT/JP02/08998
(43) Date of publication of application: 01.07.2009
(62) Divisional of application: 03794237.2
(73) Proprietor: Chugai Seiyaku Kabushiki Kaisha, Kita-ku Tokyo 115-8543 (JP)
(72) Inventor: Ohizumi, Iwao, Tokyo 115-8543 (JP); Saito, Mikiyoshi, Tokyo 115-8543 (JP)
(74) Representative: Dempster, Robert Charles

(56) References cited:
- EP-A- 0 872 488
- JP-A- 1 047 390
- JP-A- 7 076 600
- US-A- 4 965 198
- GONZALEZ A D ET AL: "OCI-5/GPC3, A GLYPICAN ENCODED BY A GENE THAT IS MUTATED IN THE SIMPSON-GOLABI-BEHMEL OVERGROWTH SYNDROME, INDUCES APOPTOSIS IN A CELL LINE-SPECIFIC MANNER" THE JOURNAL OF CELL BIOLOGY, ROCKEFELLER UNIVERSITY PRESS, US, vol. 141, no. 6, 15 June 1998 (1998-06-15), pages 1407-1414, XP002927346 ISSN: 0021-9525
- FILMUS J ET AL: "Glypicans: proteoglycans with a surprise" JOURNAL OF CLINICAL INVESTIGATION, AMERICAN SOCIETY FOR CLINICAL INVESTIGATION, US, vol. 108, no. 4, 1 August 2001 (2001-08-01), pages 497-501, XP002350773 ISSN: 0021-9738
- CAPPURO M I ET AL: "Overexpression of glypican-3 human hepatocellular carcinomas determined by immunohistochemistry using a monoclonal antibody" PROCEEDINGS OF THE ANNUAL MEETING OF THE AMERICAN ASSOCIATION FOR CANCER RESEARCH, NEW YORK, NY, US, vol. 43, March 2002 (2002-03), page 219, XP002957172 ISSN: 0197-016X
- FILMUS J: "Glypicans in growth control and cancer" GLYCOBIOLOGY, IRL PRESS,, GB, vol. 11, no. 3, March 2001 (2001-03), pages 19R-23R, XP002956385 ISSN: 0959-6658
- MIDORIKAWA Y ET AL: "Glypican-3 up-regulated in hepatocellular carcinoma, inhibits fibroblast grwoth factor 2 induced cell proliferation" PROCEEDINGS OF THE ANNUAL MEETING OF THE AMERICAN ASSOCIATION FOR CANCER RESEARCH, NEW YORK, NY, US, vol. 43, March 2002 (2002-03), page 11, XP002957173 ISSN: 0197-016X
- NAGATA S ET AL: "THE FAS DEATH FACTOR" SCIENCE, AMERICAN ASSOCIATION FOR THE ADVANCEMENT OF SCIENCE,, US, vol. 267, 10 March 1995 (1995-03-10), pages 1449-1456, XP002026333 ISSN: 0036-8075
- LAGE H ET AL: "Expression of a glypican-related 62-kDa antigen is decreased in hepatocellular carcinoma in correspondence to the grade of tumor differentiation" VIRCHOWS ARCHIV, SPRINGER INTERNATIONAL, BERLIN, DE, vol. 438, no. 6, 21 February 2001 (2001-02-21), pages 567-573, XP002379399 ISSN: 0945-6317
- TAKAHASHI N ET AL: "Improved generation of catalytic antibodies by MRL/MPJ-lpr/lpr autoimmune mice" JOURNAL OF IMMUNOLOGICAL METHODS, ELSEVIER SCIENCE PUBLISHERS B.V.,AMSTERDAM, NL, vol. 235, no. 1-2, 1 February 2000 (2000-02-01), pages 113-120, XP004188237 ISSN: 0022-1759
- BENDIG M M: "HUMANIZATION OF RODENT MONOCLONAL ANTIBODIES BY CDR GRAFTING", METHODS : A COMPANION TO METHODS IN ENZYMOLOGY, ACADEMIC PRESS INC., NEW YORK, NY, US, vol. 8, 1 January 1995 (1995-01-01), pages 83-93, XP002943667, ISSN: 1046-2023
- BENDIG M M ET AL: "THE HUMANIZATION OF MOUSE MONOCLONAL ANTIBODIES BY CDR-GRAFTINGS: EXAMPLES WITH ANTI-VIRAL AND ANTI-TUMOUR CELL ANTIBODIES", MONOCLONAL ANTIBODIES, XX, XX, 1 January 1993 (1993-01-01), pages 119-140, XP001024084,

### SEQUENCE LISTING

<110> CHUGAI SEIYAKU KABUSHIKI KAISHA
<120> Preparation of antibody using MRL/lpr mice
<130> P023151EPA
<140> 07006218.7 <141> 2003-09-04
<150> JP 2002-08998
   <151> 2002-09-04
<160> 6
<170> PatentIn ver. 2.1
<210> 1
   <211> 31
   <212> DNA
   <213> Artificial
<220>
   <223> Primer
<400> 1
   gatatcatgg ccgggaccgt gcgcaccgcg t 31
<210> 2
   <211> 31
   <212> DNA
   <213> Artificial
<220>
   <223> Primer -
<400> 2
   gctagctcag tgcaccagga agaagaagca c 31
<210> 3
   <211> 31
   <212> DNA
   <213> Artificial
<220>
   <223> Primer
<400> 3
   atagaattcc accatggccg ggaccgtgcg c 31
<210> 4
   <211> 31
   <212> DNA
   <213> Artificial
<220>
   <223> Primer
<400> 4
   ataggatccc ttcagcgggg aatgaacgtt c 31
<210> 5
   <211> 580
   <212> PRT
   <213> Homo sapiens
<220>
   <221> SIGNAL
   <223> (1)..(19)
<220>
   <221> SIGNAL
   <223> (562)..(580)
<400> 5
<210> 6
   <211> 579
   <212> PRT
   <213> Mus musculus
<220>
   <221> SIGNAL
   <222> (1)..(19)
<220>
   <221> SIGNAL
   <222> (561)..(579)
<400> 6
   Met Ala Gly Thr Val Arg Thr Ala Cys Leu Val Leu Ala Met Leu Leu
   1 5 10 15
   Gly Leu Gly Cys Leu Gly Gln Ala Gln Pro Pro Pro Pro Pro Asp Ala
   20 25 30
   Thr Cys His Gln Val Arg Ser Phe Phe Gln Arg Leu Gln Pro Gly Leu
   35 40 45
   Lys Trp Val Pro Glu Thr Pro Val Pro Gly Ser Asp Leu Gln Val Cys
   50 55 60
   Leu Pro Lys Gly Pro Thr Cys Cys Ser Arg Lys Met Glu Glu Lys Tyr
   65 70 75 80
   Gln Leu Thr Ala Arg Leu Asn Met Glu Gln Leu Leu Gln Ser Ala Ser
   85 90 95
   Met Glu Leu Lys Phe Leu Ile Ile Gln Asn Ala Ala Val Phe Gln Glu
   100 105 110
   Ala Phe Glu Ile Val Val Arg His Ala Lys Asn Tyr Thr Asn Ala Met
   115 120 125
   Phe Lys Asn Asn Tyr Pro Ser Leu Thr Pro Gln Ala Phe Glu Phe Val
   130 135 140
   Gly Glu Phe Phe Thr Asp Val Ser Leu Tyr Ile Leu Gly Ser Asp Ile
   145 150 155 160
   Asn Val Asp Asp Met Val Asn Glu Leu Phe Asp Ser Leu Phe Pro Val
   165 170 175
   Ile Tyr Thr Gln Met Met Asn Pro Gly Leu Pro Glu Ser Ala Leu Asp
               180                 185                 190
   Ile Asn Glu Cys Leu Arg Gly Ala Arg Arg Asp Leu Lys Val Phe Gly
           195                 200                 205
   Ser Phe Pro Lys Leu Ile Met Thr Gln Val Ser Lys Ser Leu Gln Val
       210                 215                 220
   Thr Arg Ile Phe Leu Gln Ala Leu Asn Leu Gly Ile Glu Val Ile Asn
   225                 230                 235                     240
   Thr Thr Asp His Leu Lys Phe Ser Lys Asp Cys Gly Arg Met Leu Thr
                   245                 250                 255
   Arg Met Trp Tyr Cys Ser Tyr Cys Gln Gly Leu Met Met Val Lys Pro
               260                 265                 270
   Cys Gly Gly Tyr Cys Asn Val Val Met Gln Gly Cys Met Ala Gly Val
           275                 280                 285
   Val Glu Ile Asp Lys Tyr Trp Arg Glu Tyr Ile Leu Ser Leu Glu Glu
       290                 295                 300
   Leu Val Asn Gly Met Tyr Arg Ile Tyr Asp Met Glu Asn Val Leu Leu
   305                 310                 315                     320
   Gly Leu Phe Ser Thr Ile His Asp Ser Ile Gln Tyr Val Gln Lys Asn
                   325                 330                 335
   Gly Gly Lys Leu Thr Thr Thr Ile Gly Lys Leu Cys Ala His Ser Gln
               340                 345                 350
   Gln Arg Gln Tyr Arg Ser Ala Tyr Tyr Pro Glu Asp Leu Phe Ile Asp
           355                 360                 365
   Lys Lys Ile Leu Lys Val Ala His Val Glu His Glu Glu Thr Leu Ser
       370                 375                 380
   Ser Arg Arg Arg Glu Leu Ile Gln Lys Leu Lys Ser Phe Ile Asn Phe
   385                 390                 395                     400
   Tyr Ser Ala Leu Pro Gly Tyr Ile Cys Ser His Ser Pro Val Ala Glu
                   405                 410                 415
   Asn Asp Thr Leu Cys Trp Asn Gly Gln Glu Leu Val Glu Arg Tyr Ser
               420                 425                 430
   Gln Lys Ala Ala Arg Asn Gly Met Lys Asn Gln Phe Asn Leu His Glu
           435                 440                 445
   Leu Lys Met Lys Gly Pro Glu Pro Val Val Ser Gln Ile Ile Asp Lys
       450                 455                 460
   Leu Lys His Ile Asn Gln Leu Leu Arg Thr Met Ser Val Pro Lys Gly
   465                 470                 475                     480
   Lys Val Leu Asp Lys Ser Leu Asp Glu Glu Gly Leu Glu Ser Gly Asp
                   485                 490                 495
   Cys Gly Asp Asp Glu Asp Glu Cys Ile Gly Ser Ser Gly Asp Gly Met
               500                 505                 510
   Val Lys Val Lys Asn Gln Leu Arg Phe Leu Ala Glu Leu Ala Tyr Asp
           515                 520                 525
   Leu Asp Val Asp Asp Ala Pro Gly Asn Lys Gln His Gly Asn Gln Lys
       530                 535                 540
   Asp Asn Glu Ile Thr Thr Ser His Ser Val Gly Asn Met Pro Ser Pro
   545 550 555 560
   Leu Lys Ile Leu Ile Ser Val Ala Ile Tyr Val Ala Cys Leu Phe Phe
   565 570 575
   Leu Val His

## Description

### Technical Field

Disclosed herein is a process for preparing an antibody using a nonhuman animal that develops autoimmune disease. More particularly; disclosed herein is a process for preparing an antibody, with the use of a nonhuman animal that develops autoimmune disease, against a protein having amino acid sequences that exhibit high homology between nonhuman animal and a human. An example of the protein of interest is glypican-3 (GPC-3).

### Background Art

Antibodies against human-derived proteins have been utilized in a wide range of applications; including diagnosis and treatment of diseases. The simplest process for obtaining such antibody is carried out vis administration of a human-derived protein to a nonhuman animal as an antigen. Such human-derived protein is recognized by the nonhuman animal as nonself, immune responses occur, and antibody production is induced in the animal. The autologous protein that is inherently possessed by the nonhuman animal is not recognized as nonself because of immunotolerance and thus immune responses are not induced and an antibody against such protein is not produced.

An antigen protein has an epitope (an antigenic determinant) consisting of 5 or 6 amino acid residues. An epitope is recognized in an animal to which the antigen has been administered and an antibody against this epitope is then produced. Formation of an epitope is affected by a protein conformation.

Amino acid sequences of some proteins are conserved during the process of evolution, and such amino acid sequences can exhibit high homology among different animal species. Since proteins having amino acid sequences that are highly homologous in different animal species are similar to each other in terms of their conformations, the epitope structures are also conserved. If a protein derived from a given species that has amino acid sequences highly homologous in different animal species is administered to a relevant animal of a different species, this protein is not recognized as nonself. Thus, an antibody is not produced, and an antibody cannot be easily obtained.

There are many proteins having amino acid sequences that are highly homologous between human and nonhuman animals such as a mouse. An example thereof is a protein belonging to the glypican family.

The glypican family has been reported as a new member of the heparan sulfate proteoglycans that exist on cell surfaces. Up to the present, existence of 5 types of glypican family members has been reported (glypicans 1, 2, 3, 4, and 5). The members of such family have core proteins of a uniform size (approximately 60 kDa), share specific and well-conserved cysteine sequences, and bind to the cell membrane via a glycosylphosphatidylinositol (GPI) anchor. Among them, glypican 3 (GPC-3) is known to be deeply involved in mitosis in the developmental process or regulation of the pattern thereof. Also, it has been known that the GPC-3 gene is expressed at a high level in the hepatic carcinoma cells, and it can be applied as the carcinoma marker. If an antibody against GPC-3 having such characteristics is obtained, such antibody could be effective for diagnosis and research related to carcinoma.

However, GPC-3 exhibits an extremely high homology of 94% at the amino acid level between a mouse and a human. Thus, an antibody against GPC-3 cannot be easily obtained since it is unlikely to be recognized as a foreign substance via conventional immunization in the case of the Balb/c mouse and the like. Accordingly, a process for easily and effectively preparing an antibody has been awaited concerning a protein having amino acid sequences that are highly homologous between human and nonhuman animals, including GPC-3.

EP0872488 discloses immunizing MPL lpr/lpr mice, which lack functional Fas, with Fas-ligand expressing cells in order to generate antibodies against Fas ligand.

### Disclosure of Invention

Disclosed herein is a process for preparing an antibody against a protein having amino acid sequences that are highly homologous between human and nonhuman animals such as a mouse with the use of an animal that develops autoimmune disease. Disclosed herein is a process for preparing an antibody against the GPC-3 protein exhibiting high amino acid sequence homology in a human and a mouse with the use of a mouse that develops autoimmune disease.

The present inventors have thoroughly considered and conducted studies as follows. Even in the case of a human-derived protein that has amino acid sequences that are highly homologous between human and nonhuman animals and that is less likely to produce an antibody upon administration of the protein to a nonhuman animal, administration thereof to a nonhuman animal that has developed autoimmune disease, where immune responses take place against the self, would lead to the production of an antibody against the aforementioned protein within the nonhuman animal. For example, the MRL/lpr mouse, which is a model of autoimmune disease, is known to produce an autoantibody (The genetics of autoantibody production in MRL/lpr lupus mice, Eisenberg R. A. et al., Clin Exp Rheumatol, 1989, Sep-Oct, 7 Suppl 3: S35-40; Hidden autoantibodies against common serum proteins in murine systemic lupus erythematosus. Detection by *in vitro* plaque-forming cell assay. Cohen PL. et al., J Exp Med 1985, Jun 1, 161 (6): 1587-92; Lpr and gld: single gene models of systemic autoimmunity and lymphoproliferative disease, Cohen PL. et al., Annu Rev Immunol 1991, 9, 243-69; Establishment of Monoclonal Anti-Retroviral gp 70 Autoantibodies from MRL/lpr Lupus Mice and Induction of Glomerular gp70 Deposition and Pathology by Transfer into Non-Autoimmune Mice. Nobutada Tabata, J of Virology May 2000; vol 74: 9: 4116-26). Accordingly, the present inventors have considered that the use of a mouse model of autoimmune disease, such as the MRL/lpr mouse, can lead to the efficient production of an antibody against a protein antigen exhibiting very high homology at the amino acid level between a mouse and a human, such as a mouse-derived antigen or GPC-3, as well as a protein antigen exhibiting low amino acid sequence homology in a mouse and a different species.

In order to inspect the effectiveness of the preparation of an antibody utilizing the MRL/lpr mouse, the present inventors immunized the MRL/lpr mouse and the Balb/c mouse while employing human GPC-3 as an immunogen and prepared an antibody. As a result, they have found that the MRL/Ipr mouse can provide positive wells with high OD values in amounts approximately 40 times larger, a wider variety of isotypes, and an affinity of an antibody for an antigen that is approximately 100 times higher than those provided by the Balb/c mouse. Thus, preparation of an antibody via immunization of the MRL/lpr mouse was found to be very effective in order to obtain an antibody against an antigen having very high homology at the amino acid level between a mouse and a human, such as GPC-3, as well as an antigen having low protein homology between a mouse and a different species. This has led to the completion of the present invention.

Disclosed herein is:
(1) a process for preparing an antibody against a glypican protein comprising immunizing a nonhuman animal that develops autoimmune disease with a glypican protein;
(2) a process for preparing an antibody against a glypican protein comprising immunizing an autoantibody-producing nonhuman animal with a glypican protein;
(3) the process for preparing an antibody against a glypican protein according to (1) or (2), wherein the nonhuman animal that develops autoimmune disease or the autoantibody-producing nonhuman animal is a nonhuman animal with Fas function defects;
(4) the process for preparing an antibody against a glypican protein according to (3), wherein the nonhuman animal is a mouse;
(5) the process for preparing an antibody against a glypican protein according to (4), wherein the mouse is the MRL/lpr mouse;
(6) the process for preparing an antibody against a glypican protein according to any of (1) to (5), wherein the glypican protein is glypican 3;
(7) a process for producing an antibody comprising immunizing a nonhuman animal with Fas function defects with an antigen;
(8) the process for preparing an antibody according to (7), wherein the nonhuman animal is a mouse;
(9) the process for preparing an antibody according to (8), wherein the mouse is the MRL/lpr mouse;
(10) the process for preparing an antibody according to any of (7) to (9), wherein the antigen protein exhibits high amino acid sequence homology in a human and a mouse;
(11) the process for preparing an antibody according to (10), wherein the amino acid sequence homology is 90% or higher; and
(12) the process for preparing an antibody according to (11), wherein the amino acid sequence homology is 94% or higher.

Hereafter, the present invention is described in greater detail.

The term "autoimmune disease" used in the present invention refers to a disease that is caused by an autoantibody. Diseases caused by an autoantibody include not only diseases caused solely by an autoantibody but also diseases caused by a complex of an autoantibody and other substances such as a complex of an autoantibody and a corresponding antigen. Diseases in which the existence of an autoantibody is closely related to the establishment of lesion are also included thereamong, along with diseases that are apparently induced by an autoantibody. Specific examples of autoimmune diseases include autoimmune hepatitis, autoimmune thyreoiditis, autoimmune bullous dermatosis, autoimmune inflammation of the adrenal cortex, autoimmune hemolytic anemia, autoimmune thrombocytopenic purpura, autoimmune atrophic gastritis, autoimmune neutropenia, autoimmune orchitis, autoimmune encephalomyelitis, autoimmune receptor disease, autoimmune infertility, rheumatism, Crohn's disease, systemic erythematodes, multiple sclerosis, Basedow's disease, juvenile diabetes, Addison's disease, myasthenia gravis, and phacogenic uveitis. Development of one or more autoimmune disease(s) is sufficient for the nonhuman animal that develops autoimmune disease according to the present invention. Autoimmune disease types are not particularly limited.

In the process for preparing an antibody disclosed herein, a nonhuman animal to be used for immunization can be a nonhuman animal that excessively produces autoantibodies compared with the case of a normal nonhuman animal as well as a nonhuman animal that has developed autoimmune disease.

Further, the process disclosed herein can employ a nonhuman animal that has been made to produce an autoantibody via, for example, administration of a polyclonal B cell activator such as LPS or dextran sulfate to such normal nonhuman animal that does not usually produce any autoantibody (e.g., the Balb/c mouse).

A preferable example of a nonhuman animal that develops autoimmune disease or a nonhuman animal that produces an autoantibody is a nonhuman animal that has abnormalities in its mechanism of immunoregulation. A specific example thereof is a nonhuman animal lacking Fas functions caused by a Fas gene mutation. Fas is a transmembrane protein that belongs to the NGF/TNF receptor family and is a receptor molecule that transmits an apoptosis-inducing signal. In general, when the B cell that responds to the autoantigen meets the autoantigen-specific T cell, the Fas ligand on the T cell surface binds to Fas (CD 95) on the B cell surface, and apoptosis is induced in the autoantigen-responding B cell. When there is a Fas mutation, however, this mechanism does not function. This results in the survival of the B cell that responds to the autoantigen and produces an autoantibody and the production of an excess amount of autoantibodies.

A specific example of a nonhuman animal that has Fas function defects is the MRL/lpr mouse. In general, the lpr mouse having the mutated Fas gene (the MRL/lpr mouse) develops a symptom of abnormal T cell accumulation and a systemic erythematodes-like autoimmune disease.

A specific example of a nonhuman animal that has Fas ligand defects is the MRL/gld mouse.

Mice that have Fas function defects, mice that have Fas ligand defects, and the like are commercially available. Thus, a person skilled in the art can easily obtain them.

Also, a mouse in which the expression of Fas or Fas ligand has been artificially repressed can be prepared in the following manner via a technique of gene targeting or other means.

At the outset, DNA including the exon portion of the Fas (or Fas ligand) gene is isolated from a mouse, an adequate marker gene is inserted into this DNA fragment, and a targeting vector is constructed. Thereafter, the resulting targeting vector is introduced into the mouse ES cell line via electroporation or other means, and a cell line in which homologous recombination has taken place is selected. An example of a marker gene to be inserted is preferably an antibiotic-resistant gene such as a neomycin-resistant gene. When an antibiotic-resistant gene is inserted, a cell line in which homologous recombination has taken place can be selected by simply conducting culture in a medium containing antibiotics. The resulting ES cell line can be injected into the blastodermic layer of a mouse to obtain a chimera mouse. By subjecting this chimera mouse to mating, a mouse with one of the Fas (or Fas ligand) gene pair being inactivated can be obtained. By subjecting the mouse to mating, a mouse with both of the Fas (or Fas ligand) gene pair being inactivated can be obtained.

Examples of a nonhuman animal that can be employed in the present invention include a monkey, a pig, a dog, a rat, a mouse, and a rabbit. Rodents such as a rat, mouse, or hamster are preferable, and a mouse is particularly preferable.

In the present invention, a protein can be used as an antigen. The antigen protein is highly homologous to a homolog protein of the nonhuman animal to be immunized that corresponds to the aforementioned antigen protein at the amino acid sequence level.

The term "highly homologous" refers to a homology of 90% or higher, and particularly preferably 94% or higher, in terms of the amino acid sequence. Optimal sequence alignments for determining protein homology can be computed via a variety of algorithms. Examples thereof include: the algorithm of Wilbur, W. J. and Lipman, D. J.; Proc. Natl. Acad. Sci. U.S.A., 1983, 80, 726-730; the local homology algorithm of Smith and Waterman, 1981, Adv. Appl. Math, 2: 482; the homology alignment algorithm of Needleman and Wunsch, 1970, J. Mol. Biol. 48: 443; similarity searching of Pearson and Lipman, 1988, Proc. Natl. Acad. Sci. U.S.A., 85: 2444; and computer programs for executing these algorithms (e.g., GAP, BESTFIT, FASTA, and TFASTA, Wisconsin Genetics Soft Package (Genetics Computer Group, Madison, WI, U.S.A.)). Sequence alignments can be computed using the BLAST algorithm that is described in Altschul et al., 1990, J. Mol. Biol. 215: 403-10 (with the use of the disclosed default settings). The software for performing BLAST analysis is available from the National Center for Biotechnology Information (http://www.ncbi.nlm.gov/). The BLAST algorithm begins with identifying short words of length W in the query sequence that either match or satisfy any positive-valued threshold score T when first aligned with short words of length W in the database sequence, thereby identifying high-scoring sequence pairs (HSPs). T is referred to as the neighborhood word score threshold. The initial neighborhood word hits act as seeds for initiating searches to find longer HSPs. The word hits are extended in both directions along each sequence for as far as the cumulative alignment score can be increased. Extension of the word hits in each direction are halted when any of the following parameters is complied with: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and the speed of the alignment. When comparing the nucleic acids of both chains, the BLAST program employs the word length (W) = 11 as a default, the BLOSUM 62 scoring matrix (Henikoff and Henikoff, 1992, Proc. Natl. Acad. Sci. U.S.A., 89: 10915-10919) alignment (B) = 50, the expectation (E) = 10 (it may be changed to 1, 0.1, 0.01, 0.001, or 0.0001 in other embodiments; with an E value that is much higher than 0.1, it is impossible to identify sequences that are functionally similar, but word hits can be effectively determined by using much lower significance, i.e., an E value between 0.1 and 10), M = 5, and N = 4 for shorter similar regions. Concerning protein comparison, the BLASTP can be used with the utilization of the following defaults: G = 11 (a cost required for providing a gap); E = 1(a cost required for expanding the gap); E = 10 (expectation, accidental 10 hits having a score equivalent to or superior to the normalized alignment score S is expected in a database having the size same as that of the target of searching; searching stringency can be altered by increasing or decreasing the E value); and W = 3 (word size: the default is 11 in relation to BLASTN and 3 in relation to other blast programs).

The BLOSUM matrix assigns a probability score for each position in an alignment, and such assignment is based on the frequency with which the substitution is known to occur among consensus blocks within related proteins. The BLOSUM62 (gap existence cost = 11; per residue gap cost = 1; λ ratio = 0.85) substitution matrix is used by default in BLAST 2.0. A variety of other matrices may also be used instead of BLOSUM62. Examples of alternatives to BLOSUM62 include PAM30 (9,1,0.87), PAM70 (10,1,0.87), BLOSUM80 (10,1,0.87), BLOSUM62 (11,1,0.82), and BLOSUM45 (14,2,0.87). One measure of a statistical analysis of the similarity between two sequences provided by the BLAST algorithm is the smallest sum probability (P(N)), which provides an indication of the probability that a match between two nucleotide or amino acid sequences would occur by chance. In the other embodiment of the present invention, a nucleotide or amino acid sequence is considered to be substantially identical to a reference sequence if the smallest sum probability thereof in comparison with the reference sequence is less than about 1, preferably less than about 0.1, more preferably less than 0.01, and most preferably less than about 0.001.

An example of a highly homologous protein can be taken from the glypican family. The glypican family is constituted by glypican 1, glypican 2, glypican 3, glypican 4, glypican 5, and the like (Trends in Glycoscience and Glycotechnology, vol. 10, No. 52, March 1998, pp. 145-152). Among the members of the glypican family, a glypican-3 protein is particularly preferable.

An antibody may be polyclonal or monoclonal, and a monoclonal antibody is preferable.

Animals are immunized with the aid of a sensitizing antigen in accordance with a conventional technique. An example of a general immunization involves intraperitoneal or hypodermic injection of the sensitizing antigen into a mammal. Specifically, the sensitizing antigen is diluted to an adequate amount and suspended with the aid of phosphate-buffered saline (PBS), physiological saline, or the like, and an adequate amount of a common adjuvant, such as Freund's complete adjuvant, is mixed therewith, if necessary. The mixture is emulsified, and the resultant is then administered to a mammal several times every 4 to 21 days. An adequate carrier can be used at the time of immunization with the sensitizing antigen. When a partial peptide having a particularly small molecular weight is used as a sensitizing antigen, immunization is preferably carried out by allowing such peptide to bind to a carrier protein, such as albumin or keyhole limpet hemocyanin.

Thus, a mammal is immunized, elevation of the antibody level to a desired level in the serum is confirmed, and the immunized cells are sampled from the mammal, followed by cell fusion. An example of a particularly preferable immunized cell is a spleen cell.

A myeloma cell of a mammal is used as a parent cell to which the aforementioned immunized cell is to be fused. A variety of known cell lines are preferably used regarding such myeloma cell. Examples thereof include P3 (P3x63Ag8.653) (J. Immnol., 1979, 123, 1548-1550), P3x63Ag8U.1 (Current Topics in Microbiology and Immunology, 1978, 81, 1-7), NS-1 (Kohler. G. and Milstein, C. Eur. J. lmmunol., 1976, 6, 511-519), MPC-11 (Margulies. D.H. et al., Cell, 1976, 8, 405-415), SP2/0 (Shulman, M. et al., Nature, 1978, 276, 269-270), FO (de St. Groth, S. F. et al., J. Immunol. Methods, 1980, 35, 1-21), S194 (Trowbridge, I. S. J. Exp. Med., 1978, 148, 313-323), and R210 (Galfre, G. et al., Nature, 1979, 277, 131-133).

When the MRL/lpr mouse is selected as an animal to be immunized with an antigen, any of the aforementioned myeloma cells can be employed.

Cell fusion between the aforementioned immunized cell and the myeloma cell can be basically realized via a conventional technique, such as the method of Kohler and Milstein (Kohler, G. and Milstein, C., Methods Enzymol., 1981, 73, 3-46).

More specifically, such cell fusion may be implemented in a common nutrient medium, for example, in the presence of a cell-fusion accelerator. Examples of a cell-fusion accelerator include polyethylene glycol (PEG) and hemagglutinating virus of Japan (HVJ). Further, an adjuvant, such as dimethyl sulfoxide, can be added in order to enhance the fusion efficiency, if necessary.

The ratio of the immunized cells to be used relative to the myeloma cells can be arbitrarily determined. For example, the quantity of the immunized cells is preferably 1 to 10 times that of the myeloma cells. Examples of a medium that can be used for the aforementioned cell fusion include RPMI 1640 medium and MEM medium, which are suitable for proliferating the myeloma cells, and are common media for such type of cell culture in combination. A blood serum adjuvant such as fetal calf serum (FCS) can also be used.

Cell fusion is realized in the following manner. Given quantities of the immunized cells and myeloma cells are thoroughly mixed in the medium, and a PEG solution that has been previously heated to approximately 37°C (e.g., a solution with an average molecular weight of approximately 1,000 to 6,000) is added thereto, generally at concentrations of 30% to 60% (w/v), followed by mixing. Thus, a fused cell (a hybridoma) of interest can be formed. Subsequently, an adequate medium is successively added, and the mixture is centrifuged to remove the supernatant. This procedure is repeated to remove a cell-fusion accelerator and other substances that are not preferable for hybridoma growth.

The thus obtained hybridoma is selected via culture in a common selection medium, such as HAT-medium (a medium containing hypoxanthine, aminopterin, and thymidine). Culture in the HAT-medium is continued for a time period that is long enough for cells other than the hybridoma of interest (nonfusion cells) to die (for several days to several weeks, in general). Subsequently, a conventional technique of limiting dilution is performed for screening and single cloning of a hybridoma that produces an antibody of interest.

The antibody of interest may be screened for and subjected to single cloning via a technique based on known antigen-antibody reactions. For example, an antigen is bound to a carrier such as polystyrene beads, a commercialized 96-well microtiter plate, or the like and then allowed to react with the culture supernatant of the hybridoma. After the carrier is washed, an enzyme-labeled secondary antibody and the like are allowed to react therewith. Thus, it can be determined whether or not the culture supernatant contains the antibody of interest that reacts with the sensitizing antigen. A hybridoma that produces an antibody of interest can be cloned by a technique of limiting dilution or by other means. In such a case, the N-terminal peptide of GPC-3 or a fragment thereof may be employed as the antigen for screening. Further, artificially modified gene recombinant antibodies such as a chimera antibody or a humanized antibody, an antibody fragment, a modified antibody, and the like can be prepared from the antibody obtained by the process according to the present invention.

The antibody gene is cloned from the hybridoma that was obtained by the process according to the present invention, the cloned gene is incorporated into an adequate vector, and the resultant is introduced into a host. Thus, a recombinant antibody can be prepared (see, for example, Vandamme, A. M. et al., Eur. J. Biochem. 1990, 192, 767-775).

Specifically, mRNA that encodes a variable (V) region of an antibody is isolated from a hybridoma that produces an antibody. mRNA can be isolated via a conventional technique. For example, total RNA is prepared via guanidine ultracentrifugation (Chirgwin, J. M. et al., Biochemistry, 1979, 18, 5294-5299) or the AGPC method (Chomczynski, P. et al., Anal. Biochem., 1987, 162, 156-159), and mRNA of interest is prepared using the mRNA Purification Kit (Pharmacia) or the like. mRNA can be directly prepared with the use of the QuickPrep mRNA Purification Kit (Pharmacia).

cDNA of the antibody V-region is synthesized from the obtained mRNA using a reverse transcriptase. cDNA is synthesized using, for example, the AMW Reverse Transcriptase First-strand cDNA Synthesis Kit (Seikagaku Kogyo). cDNA can be synthesized or amplified via, for example, the 5'-RACE method with the use of a 5'-Ampli Finder RACE kit (Clontech) or PCR (Frohman, M. A. et al., Proc. Natl. Acad. Sci. U.S.A., 1988, 85, 8998-9002; Belyavsky, A. et al., Nucleic Acids Res., 1989, 17, 2919-2932).

The DNA fragment of interest is purified from the resulting PCR product and then ligated to vector DNA. Further, a recombinant vector is prepared therefrom and then introduced into *E. coli* or the like to select a colony, thereby preparing a recombinant vector of interest. The nucleotide sequence of DNA of interest is then confirmed via a conventional technique, such as the dideoxynucleotide chain termination method.

After DNA encoding the V-region of the antibody of interest is obtained, the obtained DNA is incorporated into an expression vector comprising DNA encoding a constant (C) region of the antibody of interest.

The antibody gene is incorporated into an expression vector so as to be expressed in an expression-controlled region, for example, under the control of an enhancer or promoter. Subsequently, a host cell is transformed with the aid of this expression vector to express the antibody.

Expression of the antibody gene may be realized by separately incorporating DNA that encodes the antibody heavy (H) chain or light (L) chain into an expression vector to simultaneously transform a host cell. Alternatively, DNA that encodes the H chain and the L chain may be incorporated into a single expression vector to transform a host cell (WO 94/11523).

A transgenic animal can be employed as well as the aforementioned host cell in order to produce a recombinant antibody. For example, the antibody gene is inserted into the middle of a gene that encodes a protein inherently produced in milk (e.g., goat β casein) and is prepared as a fusion gene. A DNA fragment that contains a fusion gene into which the antibody gene has been inserted is injected into a goat germ, and this germ is introduced into a female goat. An antibody of interest is obtained from milk produced by a transgenic goat that is born from the goat that had received the aforementioned germ or an offspring thereof. Hormones may be administered to the transgenic goat, if necessary, in order to increase the amount of milk containing the antibody of interest produced from the transgenic goat (Ebert, K. M. et al., Bio/Technology, 1994, 12, 699-702).

An artificially modified gene recombinant antibody, such as a chimera antibody or a humanized antibody, can be produced via a conventional technique.

A chimera antibody can be obtained by ligating the thus obtained DNA that encodes the V region of the antibody to DNA that encodes the human antibody C region, incorporating the ligation product into an expression vector, and introducing the resultant into a host cell to produce a chimera antibody.

A humanized antibody is also referred to as a "reshaped human antibody." This antibody is prepared by implanting a complementarity-determining region (CDR) of a nonhuman mammal, such as a mouse antibody, into the CDR of a human antibody. A common technique of gene recombination therefor is also known (see European Patent Publication EP 125023, WO 96/02576).

Specifically, a DNA sequence that is designed to have CDR of the nonhuman animal-derived antibody (e.g., a mouse antibody) obtained by the process according to the present invention to be ligated to a framework region (FR) of a human antibody is synthesized by PCR using several oligonucleotides having overlapping regions at the terminal regions of both CDR and FR as primers (see the method disclosed in WO 98/13388).

The framework region of the human antibody ligated via CDR is selected based on the fact that a complementarity-determining region forms a good antigen-binding site. According to need, amino acids in the framework region of the variable region of the antibody may be substituted, in order for the complimentarity-determining region of the reshaped human antibody to form an adequate antigen-binding site (Sato, K. et al., Cancer Res., 1993, 53, 851-856).

The C region of the human antibody is used in the C region of the chimera antibody and that of the humanized antibody. For example, Cγ1, Cγ2, Cγ3, or Cγ4 can be used for the H chain, and Cκ or Cλ can be used for the L chain. In order to improve the stability of an antibody or that for the production thereof, a human antibody C region may be modified.

A chimera antibody is constituted by a variable region of a nonhuman animal-derived antibody and a constant region of a human-derived antibody. In contrast, a humanized antibody is constituted by the complementarity-determining region of a nonhuman animal-derived antibody and the framework region and the C region of the human-derived antibody. Since the humanized antibody exhibits deteriorated antigenecity in the human body, administration thereof to a human is useful when treatment or the like is intended.

An antibody fragment or a modification product thereof can be prepared from the antibody obtained by the process according to the present invention. Examples of an antibody fragment include Fab, F(ab')2, Fv, Fab/c having a Fab and a complete Fc, and single-chain Fv (scFv) to which Fv of the H or L chain has been ligated with the aid of an adequate linker. Specifically, an antibody is processed with an enzyme such as papain or pepsin to generate an antibody fragment. Alternatively, a gene that encodes any of the aforementioned antibody fragments is constructed, the resulting gene is introduced into an expression vector, and the resultant is then allowed to express in an adequate host cell (see, for example, Co, M.S. et al., J. Immunol., 1994, 152, 2968-2976; Better, M. & Horwitz, A. H. Methods in Enzymology, 1989, 178, 476-496; Academic Press, Inc., Plueckthun, A. & Skerra, A. Methods in Enzymology, 1989, 178, 476-496; Academic Press, Inc., Lamoyi, E., Methods in Enzymology, 1989, 121, 652-663; Rousseaux, J. et al., Methods in Enzymology, 1989, 121, 663-669; Bird, R. E. et al., TIBTECH, 1991, 9, 132-137).

scFv is obtained by ligating the H chain V region to the L chain V region of the antibody. In this scFv, the H chain V region is ligated to the L chain V region through a linker, and preferably through a peptide linker (Huston, J. S. et al., Proc. Natl. Acad. Sci. U.S.A., 1988, 85, 5879-5883). An example of a peptide linker that ligates the V regions is a single-stranded peptide consisting of 12 to 19 amino acid residues.

DNA that encodes scFv is obtained in the following manner. In DNA that encodes the antibody H chain or the H chain V region and DNA that encodes the L chain or L chain V region, a DNA portion that encodes all or some of the relevant amino acid sequences is employed as a template, and a primer pair that specifies the both terminuses thereof is used to amplify the sequence by PCR. Subsequently, DNA that encodes a peptide linker portion and a primer pair that is designated to have each end ligated to the H chain or the L chain are amplified in combination.

Once DNA that encodes scFv is prepared, an expression vector containing such DNA and a host that has been transformed with the aid of such expression vector can be obtained in accordance with a conventional technique. Also, use of such host enables the production of scFv in accordance with a conventional technique.

Examples of modified antibodies include antibodies that are bound to a variety of molecules such as cytotoxic substances (e.g., a chemotherapeutant, radioactive substance, or cell-derived toxin) or labeling substances (e.g., a fluorescent dye, enzyme, coenzyme, chemiluminescent substance, or radioactive substance). Such modified antibodies can be obtained by chemically modifying the antibody obtained by the process according to the present invention. A technique of antibody modification has been already established in the art.

Further, a bispecific antibody can be prepared from the antibody obtained by the process according to the present invention. Examples of a bispecific antibody include: one that has an antigen-binding site that recognizes different epitopes on the same antigen molecule; one in which one of the antigen-binding sites recognizes an antigen and another antigen-binding site recognizes other substances such as a labeling substance; and one in which one of the antigen-binding sites recognizes the first antigen and another antigen-binding site recognizes other antigen, i.e., the second antigen. A bispecific antibody can be prepared by binding the HL pairs of two kinds of antibodies. Alternatively, it can be obtained by fusing hybridomas that independently produce different monoclonal antibodies to prepare bispecific antibody-producing fusion cells. Furthermore, a bispecific antibody can be prepared by a gene engineering technique.

The thus constructed antibody gene can be expressed and obtained by a conventional technique. In mammalian cells, gene expression can be realized by functionally binding a commonly used useful promoter, the antibody gene to be expressed, and poly A signal to the 3'-downstream. An example of a promoter/enhancer is the human cytomegalovirus immediate-early promoter/enhancer.

Examples of other promoters/enhancers that can be used for expression of the antibody gene include viral promoters/enhancers, such as retrovirus promoters/enhancers, polyoma virus promoters/enhancers, adenovirus promoters/enhancers, simian virus 40 (SV 40) promoters/enhancers, and mammalian cell-derived promoters/enhancers, such as human elongation factor la (HEF1a) promoters/enhancers.

Gene expression can be easily realized by the method of Mulligan et al. (Nature, 1979, 277, 108) when the SV 40 promoter/enhancer is used and by the method of Mizushima et al. when the HEF1a promoter/enhancer is used (Nucleic Acids Res., 1990, 18, 5322).

In the case of *E. coli,* an antibody gene can be expressed therein by functionally binding a common useful promoter, a signal sequence for antibody secretion, and the antibody gene to be expressed. Examples of a promoter include lacZ promoter and araB promoter. The gene can be expressed by the method of Ward et al. (Nature 1098, 341, 544-546; FASEB J., 1992, 6, 2422-2427) when lacZ promoter is used and by the method of Better et al. (Science, 1998, 240, 1041-1043) when araB promoter is used.

When the antibody is generated in the *E. coli* periplasm, a pelB signal sequence (Lei, S. P. et al., J. Bacteriol., 1987, 169, 4379) may be used as the signal sequence for antibody secretion. After the antibody generated in the periplasm is separated, the structure of the antibody is adequately refolded and then used.

Examples of the origins of replication include those derived from SV 40, polyoma virus, adenovirus, and bovine papilloma virus (BPV). Further, the expression vector can contain, for example, the aminoglycoside phosphotransferase (APH) gene, the thymidine kinase (TK) gene, the *E. coli* xanthine-guanine phosphoribosyl transferase (Ecogpt) gene, and the dihydrofolate reductase (dhfr) gene as a selection marker in order to increase the number of genes copied in the host cell line.

Any expression system, for example, eukaryotic or prokaryotic systems, can be used to produce an antibody. Examples of eukaryotic cell lines include the established mammalian cell lines, insect cell lines, and animal cell lines such as filamentous fungus cell lines and yeast cell lines. Examples of prokaryotic cell lines include bacterial cell lines such as *E. coli* cell lines.

An antibody is preferably expressed in a mammalian cell, such as a CHO, COS, myeloma, BHK, Vero, or HeLa cell.

Subsequently, the transformed host cell can be cultured *in vitro* or *in vivo* to obtain the antibody of interest. A host cell is cultured in accordance with a conventional technique. For example, DMEM, MEM, RPMI 1640, or IMDM medium can be used, and a blood serum adjuvant such as fetal calf serum (FCS) can also be used in combination therewith.

The thus expressed and generated antibody can be separated from a cell or host animal and then purified to homogeneity. The antibody can be separated and purified with the use of an affinity column. Examples of affinity column chromatography utilizing a protein A column include Hyper D, POROS, and Sepharose F.F. (Pharmacia). In addition, techniques of separation and purification that are employed for a common protein may be employed without particular limitation. For example, the antibody can be separated and purified by adequately selecting and combining techniques of column chromatography other than the aforementioned affinity column chromatography, filtration, ultrafiltration, salting-out, dialysis, and the like (Antibodies A Laboratory Manual, Ed Harlow, David Lane, Cold Spring Harbor Laboratory, 1988).

### Brief Description of the Drawings

Fig. 1 shows comparison of amino acid sequences between human GPC-3 and mouse GPC-3.
Fig. 2 shows a frequency table showing the results of primary screening of hybridomas.
Fig. 3 shows a detail of the monoclonal antibody isotypes of the 47 obtained clones.
Fig. 4 shows the results of the kinetic analysis of the anti-GPC-3 antibody using BIAcore.

### Preferred Embodiments of the Invention

The present invention is hereafter described in greater detail with reference to the following examples, although the technical scope of the present invention is not limited to these examples.

In the examples described in the specification of the present application, the following materials were employed.

### Materials

As expression vectors of soluble GPC-3 and a soluble GPC-3 core protein, pCXND2 and pCXND3, which were prepared by separately incorporating the DHFR gene and the neomycin-resistant gene into pCAGGS, were used.

DXB11 cells were purchased from ATCC, and 5% FBS (GIBCO BRL CAT# 10099-141, LOT# A0275242)/Minimum Essential Medium Alpha medium (αMEM(+)) (GIBCO BRL CAT# 12571-071)/1% Penicillin-Streptomycin (GIBCO BRL CAT# 15140-122) was used for culture. In order to select the expressed cell using DXB11, 500 µg/ml Geneticin (GIBCO BRL CAT# 10131-027)/5% FBS/αMEM without ribonucleosides and deoxyribonucleosides (GIBCO BRL CAT# 12561-056) (αMEM(-))/PS or a mixture prepared by adding MTX thereto to a final concentration of 25 nM was used.

The obtained hybridomas were cultured in 10% FBS/RPMI 1640/1 x HAT media supplement (SIGMA CAT# H-0262)/0.5 x BM-Condimed H1 Hybridoma cloning supplement (Roche CAT# 1088947).

### Method

### [Preparation of soluble human GPC-3]

A full-length cDNA encoding human GPC-3 was amplified by PCR using, as a template, the 1 st strand cDNA prepared from a colon cancer cell line (CaCo2) in accordance with a conventional technique, the upper primer (5'- GAT ATC ATG GCC GGG ACC GTG CGC ACC GCG T -3' (SEQ ID NO: 1)), and the lower primer (5'- GCT AGC TCA GTG CAC CAG GAA GAA GAA GCA C -3' (SEQ ID NO: 2)). Plasmid DNA containing this full-length human GPC-3 cDNA was used to construct plasmid DNA that expresses soluble GPC-3 cDNA. PCR was carried out using the lower primer ((5'- ATA GAA TTC CAC CAT GGC CGG GAC CGT GCG C -3' (SEQ ID NO: 3)) designed to lack a C-terminal hydrophobic region (a region between amino acids 564 and 580) and the upper primer (5'- ATA GGA TCC CTT CAG CGG GGA ATG AAC GTT C -3' (SEQ ID NO: 4)) to which the EcoRI recognition sequence and the Kozak sequence have been added. The resulting PCR fragment (1,711 bp) was cloned into pCXND2-Flag. The prepared expression plasmid DNA was introduced into the CHO DXBI11 cell line, and selection was performed in 500 µg/ml of geneticin to obtain a CHO cell line that expresses high levels of soluble GPC-3.

The CHO cell line that expresses high levels of soluble GPC-3 was subjected to mass-culture in a 1,700-cm²-roller bottle, and the culture supernatant was recovered, followed by purification. The culture supernatant was charged onto the DEAE Sepharose Fast Flow (Amersham CAT# 17-0709-01), washed, and then eluted with the aid of a buffer containing 500 mM NaCl. Affinity purification was then carried out using the anti-FLAG M2-agarose affinity gel (Sigma, Cat # A-2220). Elution was carried out using 200 µg/ml FLAG peptide. After the concentration with the Centriprep-10 (Millipore CAT# 4304), gel filtration was carried out using the Superdex 200 HR 10/30 (Amersham CAT# 17-1088-01) to remove the FLAG peptide. Finally, concentration was carried out using the DEAE Sepharose Fast Flow column, and elution was simultaneously carried out using PBS that does not contain Tween 20 (containing 500 mM NaCl) to replace buffers.

### [Preparation of soluble human GPC-3 core protein]

cDNA was prepared using wild type human GPC-3 cDNA as a template and substituting Ser 495 and 509 with Ala via assembly PCR. In this case, a primer was designed to have a His-tagged C terminus, and the obtained cDNA was cloned into a pCXND3 vector. The prepared expression plasmid DNA was introduced into the DXB11 cell line, and selection was carried out using 500 µg/ml geneticin to obtain a CHO cell line that expresses high levels of soluble GPC-3 core proteins.

The CHO cell line that expresses high levels of soluble GPC-3 core proteins was subjected to mass-culture in a 1,700-cm² roller bottle, and the culture supernatant was recovered, followed by purification. The culture supernatant was charged onto the Q Sepharose Fast Flow (Amersham CAT# 17-0510-01), washed, and then eluted with the aid of a phosphate buffer containing 500 mM NaCl. Affinity purification was then carried out using Chelating Sepharose Fast Flow (Amersham CAT# 17-0575-01). Gradient elution was carried out using 10 to 150 mM imidazole. Finally, concentration was carried out using the Q Sepharose Fast Flow, and elution was carried out using a phosphate buffer containing 500 mM NaCl.

### [Preparation of anti-GPC-3 antibody]

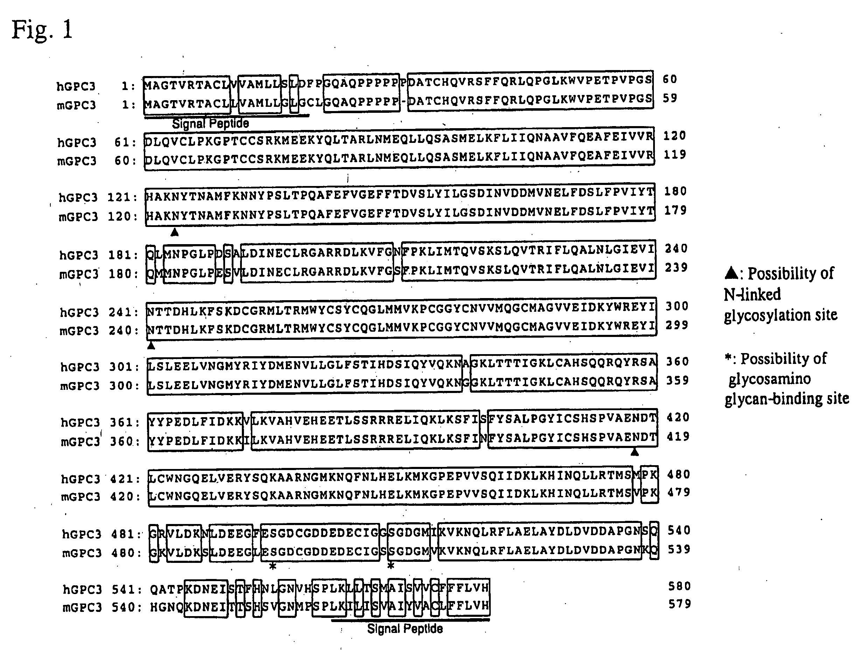

Human GPC-3 and mouse GPC-3 are highly homologous, and the level of homology is as high as 94% at the amino acid level. A comparison of amino acid sequences between human GPC-3 and mouse GPC-3 is shown in Fig. 1. In the sequences shown in Fig. 1, a portion indicated by a black triangle represents a site that may be an N-linked glucosylation site, and a portion indicated by an asterisk represents a site to which glycosaminoglycan may bind. Accordingly, it was deduced that an antibody was difficult to obtain via common immunization of a mouse. However, the MRL/lpr mouse that is known as a mouse model of autoimmune disease produces a variety of autoantibodies. This indicates that immunization of the MRL/lpr mouse can result in the production of an antibody against an antigen that is highly homologous in a human and a mouse, such as GPC-3, as well as an antigen with low protein homology in a mouse and other species. In order to verify the usefulness of the preparation of an antibody utilizing the MRL/lpr mouse, antibody production via immunization of the MRL/lpr mouse and the Balb/c mouse was compared and examined.

### Immunization and preparation of hybridoma

A soluble GPC-3 protein to which heparan sulfate had been added was used as an immunogen. In accordance with a conventional technique, 5 Balb/c mice (female, 6 weeks old, Charles River Japan) and 7 MRL/lpr mice (male, 7 weeks old, Charles River Japan) were subjected to immunization. Specifically, the immune protein was prepared in amounts of 100 µg/mouse for the primary immunization, the prepared immuno protein was emulsified with the use of FCA (Freund's complete adjuvant (H37Ra), Difco (3113-60), Becton Dickinson (cat# 231131)), and the resulting emulsion was administered hypodermically. The immunogen prepared in amounts of 50 µg/mouse was emulsified with FIA (Freund's incomplete adjuvant, Difco (0639-60), Becton Dickinson (cat# 263910)), and the resulting emulsion was administered hypodermically 2 weeks thereafter. Thereafter, booster immunizations were carried out 5 times in total at intervals of 1 week. At the final immunization, the immunogen was diluted with PBS to 50 µg/mouse, and the diluted immunogen was administered in the caudal veins. After the saturation of the antibody titer in the blood serum against GPC-3 was confirmed via ELISA using an immunoplate coated with 1 µg/ml soluble GPC-3 core proteins at 100 µl/well, the Balb/c mouse No. 2 and the MRL/lpr mouse No. 6 were subjected to final immunization, and the mouse myeloma cells P3U1 and the mouse spleen cells were mixed in accordance with a conventional technique to conduct cell fusion using PEG1500 (Roche Diagnostics, cat# 783 641). Since the number of mononuclear cells derived from the MRL/lpr mouse spleen is larger than that of the Balb/c mouse, the Balb/c-derived hybridomas were inoculated on ten 96-well culture plates, and the MRL/lpr-derived hybridomas were inoculated on twenty 96-well culture plates. Selection was initiated in HAT medium on the day following the fusion, the culture supernatant was collected 10 days and 14 days after the fusion, and ELISA screening was then carried out. As with the case of the aforementioned assay of antibody titer, ELISA screening was carried out using immunoplates coated with 1 µg/ml of soluble GPC-3 core proteins at 100 µl/well.

### Screening

In general, IgG3 and IgM are known as isotypes that have potent binding activities with complements and that are capable of inducing CDC activity. Feasibility of screening without missing IgG3 and IgM at the primary screening is very useful when cancer treatment is intended, as is the case of the anti-GPC-3 antibody. Thus, two-phase screening that recovers IgG3 and IgM as well as IgG1, IgG2a, and IgG2b, was carried out by changing the secondary antibody. More specifically, an alkaline phosphatase-labeled anti-mouse IgG(γ) antibody (Zymed, Cat No. 62-6622) was used as a secondary antibody to develop a color, thereby obtaining IgG1, IgG2a, and IgG2b at the first phase. After the plate had been thoroughly washed, the biotin-labeled anti-IgG3 antibody (Monosan, Cat No. Mon 5056B) and the horseradish peroxidase-labeled anti-IgM antibody (Zymed, Cat No. 62-6820) were then used to redevelop a color at the second phase. Thus, screening was carried out by selectively obtaining IgG3 and IgM.

The Balb/c mouse No. 2 and the MRL/lpr mouse No. 6 were independently subjected to cell fusion, and positive wells were selected via ELISA screening that employed the GPC-3 core protein as an antigen. The positive wells were spread on a 24-well plate and then cloned by limiting dilution (a positive well is sowed on a 96-well plate).

### Antibody purification

The IgG1, IgG2a, and IgG2b antibodies were purified from the obtained culture supernatant using a Protein G column (Hi-Trap Protein G HP, Amersham, CAT# 17-0404-01), and the IgM antibody was purified therefrom using a Protein L column. Specifically, IgG was purified using the Hi-Trap Protein G HP (Amersham, CAT# 17-0404-01). The culture supernatant of hybridomas was directly charged onto the column, washed with a binding buffer (20 mM sodium phosphate, pH 7.0), and then eluted with an elution buffer (0.1M glycin-HCL, pH 2.7). Elution was carried out in a tube containing a neutralization buffer (1M Tris-HCl, pH 9.0) and the elution product was neutralized immediately thereafter. After the antibody fractions were pooled, dialysis was carried out in 0.05% Tween 20/PBS overnight, and buffers were replaced. NaN₃ was added to the purified antibody to a concentration of 0.02%, and the resultant was stored at 4°C.

In contrast, IgM was purified using the ImmunoPure Immobilized Protein L (Pierce, Cat# 20510). The culture supernatant of hybridomas was directly charged onto the column, washed with a binding buffer (100 mM sodium phosphate, pH 7.2, 150 mM NaCl), and then eluted with an elution buffer (0.1M glycin-HCl, pH 2.5). After the elution, procedures were performed as in the case of IgG, and the resultant was stored at 4°C.

### [Analysis of the anti-GPC-3 antibody isotype]

Isotyping of the anti-glypican-3 antibody was carried out using the ImmunoPure Monoclonal Antibody Isotyping Kit II (Pierce, CAT# 37502) in accordance with the instructions attached to the kit.

### [Kinetic analysis of the anti-GPC-3 antibody using BIAcore]

### Preparation of soluble GPC-3 core protein chip

A soluble GPC-3 core protein was subjected to buffer replacement via gel filtration with 10 mM Na acetate (pH 5.0). The soluble GPC-3 core protein (approximately 10 µg) that had been subjected to buffer replacement was amine-coupled to the sensor chip CM5 (BIAcore, BR-1000-14) in accordance with the technique described in the amine coupling kit (BIAcore, BR-1000-50). Through this procedure, approximately 3,000 RU of soluble GPC-3 core proteins were immobilized on the CM5 chip.

### Kinetic analysis of the anti-GPC-3 antibody

The kinetic analysis described below was performed using BIAcore (BIAcore, BIACORE 2000). Each anti-GPC-3 antibody was diluted with an HBS-EP buffer to 1.25, 2.5, 5, 10, and 20 µg/ml, respectively. The HBS-EP buffer (BIAcore, BR-1001-88) was used as a running buffer, and 40 µl of the antibody at each concentration was injected at a flow rate of 20 µl/min. A time period of 2 minutes during the injection of the antibody was designated for the association phase, the buffer was replaced with a running buffer, and a time period of 2 minutes during the injection of the running buffer was designated for the dissociation phase. After the completion of the dissociation phase, 10 µl of 10 mM glycine (pH 2.2) and 5 µl of 0.05% SDS were consecutively injected to regenerate a sensor chip.

The sensorgrams obtained via the aforementioned procedures were overwritten, and the association rate constant (Ka), the dissociation rate constant (kd), the dissociation constant (KD), and the Rmax (maximal bound amount) were calculated using the BIAevaluation (ver. 3.0).

### Results

### [Preparation ofrecombinant GPC-3]

A soluble GPC-3 protein lacking the C-terminal hydrophobic region was prepared as a material for preparing the anti-GPC-3 antibody. Plasmid DNA that expresses soluble GPC-3 was introduced into the CHO cell to construct a stable cell line. After the culture supernatant was roughly purified and concentrated on an anion exchange column, the culture supernatant was subjected to affinity purification utilizing a Flag tag added to the C-terminus. As a result of SDS polyacrylamide gel electrophoresis, a smear band of 50-300 kDa and a band of approximately 40-kDa were obtained. GPC-3 is a proteoglycan comprising a 69-kDa additional heparan sulfate sequence at its C-terminus. The smear band was considered to be GPC-3 that had been modified with heparan sulfate. As a result of amino acid sequencing, a band of approximately 40-kDa was found to originate from a N-terminal fragment of GPC-3. Thus, it was deduced that GPC-3 had undergone some kind of cleavage.

In order to eliminate an antibody against heparan sulfate via hybridoma screening, a soluble GPC-3 core protein in which Ser 495 and Ser 509, which was in a heparan sulfate additional signal sequence, had been substituted with Ala, was prepared. The CHO cell line that expresses high levels of soluble GPC-3 was constructed in the same manner as described above, and the culture supernatant was subjected to affinity purification using an His-tag. As a result of SDS polyacrylamide gel electrophoresis, 3 bands of 70-kDa, 40-kDa, and 30-kDa were obtained. As a result of amino acid sequencing, the band of 30-kDa was found to be a C-terminal fragment of GPC-3, and GPC-3 was found to have undergone some type of enzymatic cleavage between arginine 358 and serine 359. It is considered that the band of 30-kDa was not observed in heparan sulfate-added GPC-3 because the band became smeared due to the addition of heparan sulfate. It is a novel finding that GPC-3 receives enzymatic cleavage in a specific amino acid sequence, and the biological significance thereof has not yet been elucidated.

### [Preparation of anti-GPC-3 antibody]

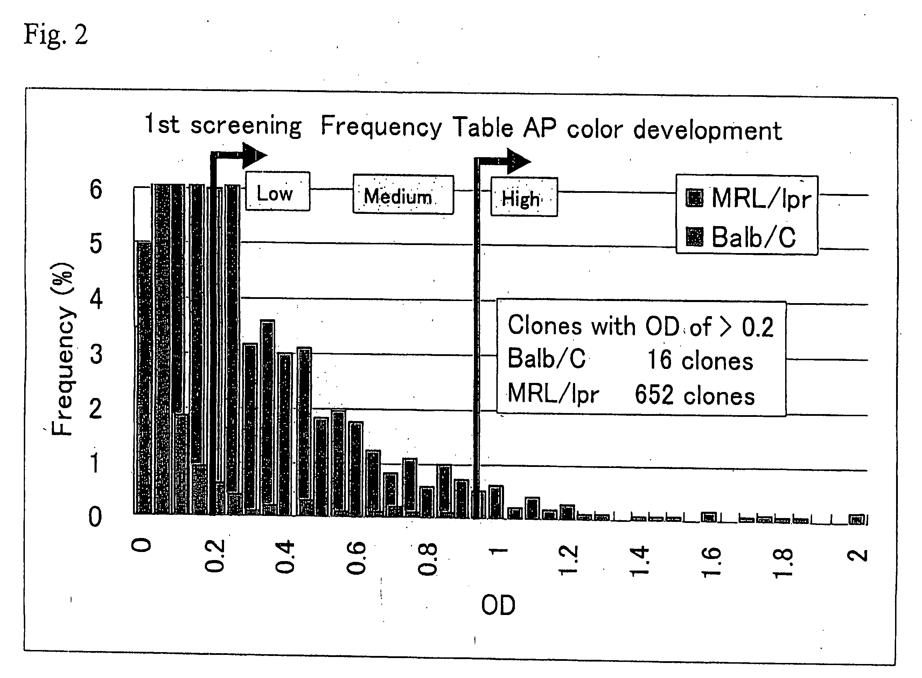

The anti-GPC-3 antibody was prepared by a hybridoma technique. Soluble GPC-3 to which purified heparan sulfate had been added was used as the immunogen. After the saturation of the antibody titer against GPC-3 in the blood serum was confirmed, mouse myeloma cells P3U1 and mouse spleen cells were subjected to cell fusion. A Balb/c mouse No. 2 and an MRL/lpr mouse No. 6 were subjected to cell fusion, and a total of 180 positive wells were selected from these two mice via ELISA screening employing the GPC-3 core protein as the immunogen. As a result, the quantity of clones indicating the OD value of 0.2 or more was 652 wells in the case of the MRL/lpr mouse and 16 wells in the case of the Balb/c mouse. The quantity of clones having high OD values (0.2 or higher) obtained from the MRL/lpr mouse was as large as approximately 40 times the figure for the Balb/c mouse. The comparison of the primary screening results of the MRL/lpr mice is shown in Fig. 2 as a frequency table.

After the primary screening, the 180 positive wells were expanded on a 24-well plate and then cloned by limiting dilution (whereby a positive well is plated on a 96-well plate). Finally, 47 clones that stably produced antibodies were established.

### [Isotype analysis of anti-GPC-3 antibody]

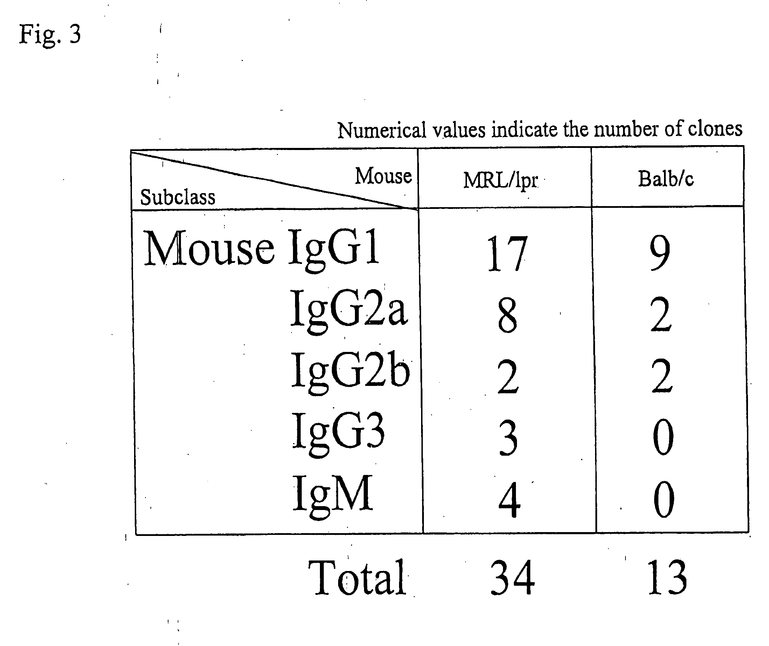

The isotypes of the 47 established clones of the anti-GPC-3 antibody were analyzed. This revealed that the Balb/c-derived antibodies were exclusively of 3 types, i.e., IgG1, IgG2a, and IgG2b. In contrast, the MRL/Ipr-derived antibodies included all the IgG subclasses, i.e., IgG1, IgG2a, IgG2b, and IgG3, and IgM. The isotypes of the 47 established clones are shown in detail in Fig. 3.

Accordingly, it was demonstrated that use of the MRL/lpr mouse could result in a wider variety of isotypes than the use of the Babl/c mouse, and its usefulness was also exhibited. Since isotypes generally having low expression frequency, such as IgG3 and IgM, could be obtained, preparation of an antibody utilizing the MRL/lpr mouse was found to be useful when preparation of an antibody having CDC activity was intended.

### [Kinetic analysis of anti-GPC-3 antibody using BIAcore]

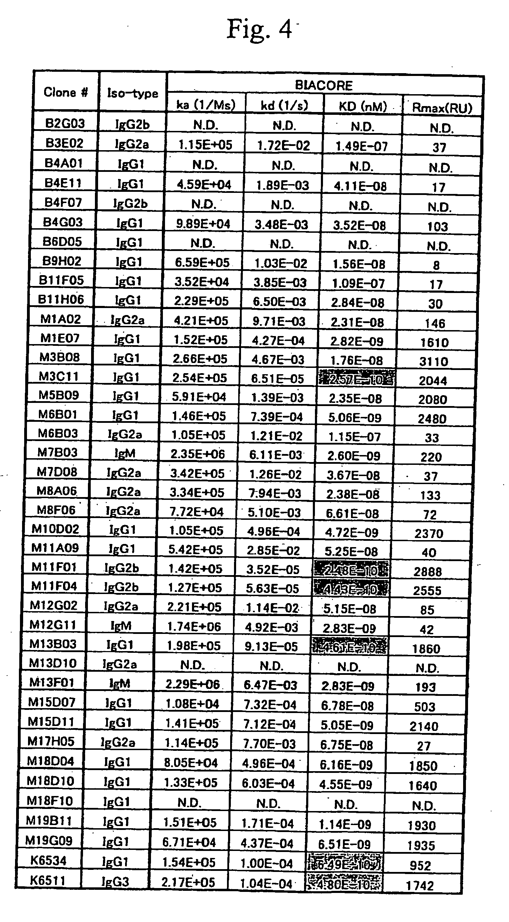

Kinetic analysis of 10 clones of the purified antibodies derived from the Balb/c mouse and 28 clones of those derived from the MRL/lpr mouse were carried out using BIAcore. The results of the kinetic analysis are shown in Fig. 4. The number of antibodies with high affinity derived from the MRL/lpr mouse was larger than that derived from the Balb/c mouse. While the dissociation constant of the Balb/c mouse-derived antibody with the highest affinity was 10⁻⁸ M, four types of MRL/lpr mouse-derived antibodies exhibited high affinity (two types of the IgG1 types and those of IgG2b types), which had the dissociation constant of the order of 10⁻¹⁰ M. Thus, the antibody obtained from the MRL/lpr mouse had affinity as high as approximately 100 times that obtained from the Balb/c mouse. This indicates the usefulness of the preparation of an antibody utilizing the MRL/lpr mouse.

### Examination and conclusion

GPC-3 exhibits an extremely high homology of 94% at the amino acid level between a mouse and a human. Thus, it can be hard to obtain an antibody via conventional immunization of a Balb/c mouse or the like. Since the MRL/lpr mouse that is a model of autoimmune disease lacks Fas-ligand functions, it is considered that apoptosis of the autoantibody-producing B cells is not induced and the mechanism of breaking the immunotolerance is active. Accordingly, use of a mouse model of autoimmune disease, such as the MRL/lpr mouse, enables the effective production of an antibody against murine antigens and antigens that are highly homologous to each other at the amino acid level between a human and a mouse, such as a mouse antigen or GPC-3, as well as antigens that have low protein homology between a mouse and an animal of different species.

Via this examination, the MRL/lpr mouse was found to be capable of providing positive wells with high OD values in amounts of approximately 40 times larger than those in the case of the Balb/c mouse, with a wider variety of isotypes and an affinity of an antibody for antigen that is approximately 100 times higher.

### Industrial Applicability

According to the foregoing description, preparation of an antibody via immunization of the MRL/lpr mouse is considered a very effective way to obtain an antibody against an antigen exhibiting very high homology at the amino acid level between a mouse and a human, such as a mouse antigen or GPC-3, as well as the antigens having low protein homology in a mouse and an animal of a different species.

Further processes are disclosed herein as defined in the following numbered paragraphs:
1. A process for producing an antibody against a glypican protein comprising immunizing a nonhuman animal that develops autoimmune disease with a glypican protein.
2. A process for producing an antibody against a glypican protein comprising immunizing an autoantibody-producing nonhuman animal with a glypican protein.
3. The process for producing an antibody against a glypican protein according to paragraph 1 or 2, wherein the nonhuman animal that develops autoimmune disease or the autoantibody-producing nonhuman animal is a nonhuman animal with Fas function defects.
4. The process for producing an antibody against a glypican protein according to paragraph 3, wherein the nonhuman animal is a mouse.
5. The process for producing an antibody against a glypican protein according to paragraph 4, wherein the mouse is the MRL/lpr mouse.
6. The process for producing an antibody against a glypican protein according to any one of paragraphs 1 to 5, wherein the glypican protein is glypican 3.
7. A process for producing an antibody comprising immunizing a nonhuman animal with Fas function defects with an antigen.
8. A process for producing an antibody according to paragraph 7, wherein the nonhuman animal is a mouse.
9. The process for producing an antibody according to paragraph 8, wherein the mouse is the MRL/lpr mouse.
10. The process for producing an antibody according to any one of paragraphs 7 to 9, wherein the antigen protein exhibits high amino acid sequence homology in a human and a mouse.
11. The process for producing an antibody according to paragraph 10, wherein the amino acid sequence homology is 90% or higher.
12. The process for producing an antibody according to paragraph 11, wherein the amino acid sequence homology is 94% or higher.

## Claims

1. A process for producing a humanized antibody comprising:
(a) immunizing a non-human animal with Fas function defects that develops autoimmune disease with an antigen that exhibits amino acid sequence homology of 90% or higher in a human and a non-human animal;
(b) obtaining an antibody from the non-human animal; and
(c) replacing a complementarity-determining region (CDR) of a human antibody with a CDR of the non-human animal antibody.

2. The process for producing a humanized antibody according to claim 1, further comprising substituting amino acids in the framework region of the variable region of the antibody.

3. The process for producing an antibody according to claim 1 or 2, wherein the nonhuman animal is a mouse.

4. The process for producing an antibody according to claim 3, wherein the mouse is the MRL/lpr mouse.

5. The process for producing an antibody according to any one of claims 1 to 4, wherein the amino acid sequence homology is 94% or higher.

## Patentansprüche

1. Verfahren zur Herstellung eines humanisierten Antikörpers, das Folgendes umfasst:
(a) Immunisieren eines nichtmenschlichen Tiers mit Fas-Funktionsdefekten, bei dem sich eine Autoimmunkrankheit entwickelt, mit einem Antigen, das eine Aminosäuresequenzhomologie von 90% beil einem Menschen und einem nichtmenschlichen Tier aufweist;
(b) Gewinnen eines Antikörpers aus dem nichtmenschlichen Tier; und
(c) Ersetzen der CDR (Complementarity-Determining Region) eines menschlichen Antikörpers mit einer CDR des nichtmenschlichen tierischen Antikörpers.

2. Verfahren zur Herstellung eines humanisierten Antikörpers nach Anspruch 1, das weiterhin Folgendes umfasst: Ersetzen von Aminosäuren in der Gerüstregion der variablen Region des Antikörpers

3. Verfahren zur Herstellung eines Antikörpers nach Anspruch 1 oder 2, wobei es sich bei dem nichtmenschlichen Tier um eine Maus handelt.

4. Verfahren zur Herstellung eines Antikörpers nach Anspruch 3, wobei es sich bei der Maus um die MRL/lpr-Maus handelt.

5. Verfahren zur Herstellung eines Antikörpers nach einem der Ansprüche 1 bis 4, wobei die Aminosäuresequenzhomologie 94% oder mehr beträgt.

## Revendications

1. Procédé de production d'un anticorps humanisé, comprenant :
(a) l'immunisation d'un animal non humain présentant des défauts de la fonction Fas, qui développe une maladive auto-immune, avec un antigène qui présente une homologie de séquence d'acides aminés de 90 % ou plus chez un humain et un animal non humain ;
(b) l'obtention d'un anticorps à partir de l'animal non humain ; et
(c) le remplacement d'une région déterminant la complémentarité (CDR) d'un anticorps humain par une CDR de l'anticorps de l'animal non humain.

2. Procédé de production d'un anticorps humanisé selon la revendication 1, comprenant en outre une substitution d'acides aminés dans la région charpente de la région variable de l'anticorps.

3. Procédé de production d'un anticorps selon la revendication 1 ou 2, dans lequel l'animal non humain est une souris.

4. Procédé de production d'un anticorps selon la revendication 3, dans lequel la souris est la souris MRL/lpr.

5. Procédé de production d'un anticorps selon l'une quelconque des revendications 1 à 4, dans lequel l'homologie de séquence d'acides aminés est de 94 % ou plus.
